(19)

Europäisches
Patentamt
European
Patent Office
Office européen
des brevets

(11) **EP 4 298 936 A2**

(12) **EUROPEAN PATENT APPLICATION**

(43) Date of publication:
**03.01.2024 Bulletin 2024/01**

(21) Application number: **23210344.0**

(22) Date of filing: **18.05.2018**

(51) International Patent Classification (IPC):
***A24F 40/53*** *(2020.01)*

(52) Cooperative Patent Classification (CPC):
**A24F 40/40; A24B 15/16; A24F 40/51; A24F 40/53;**
A24F 40/10; A24F 40/20

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.05.2017 KR 20170065550
30.10.2017 KR 20170142578
03.05.2018 KR 20180051469**

(62) Document number(s) of the earlier application(s) in
accordance with Art. 76 EPC:
**18806877.9 / 3 632 240**

(71) Applicant: **KT&G Corporation**
**Daedeok-gu**
**Daejeon 34337 (KR)**

(72) Inventor: **Lim, Hun Il**
**05555 Seoul (KR)**

(74) Representative: **Ter Meer Steinmeister & Partner**
**Patentanwälte mbB**
**Nymphenburger Straße 4**
**80335 München (DE)**

Remarks:
This application was filed on 16-11-2023 as a
divisional application to the application mentioned
under INID code 62.

(54) **AEROSOL GENERATION DEVICE HAVING CIGARETTE INSERTION DETECTION FUNCTION
AND METHOD**

(57)    Provided are an aerosol generating device for
generating an aerosol by heating a cigarette and a method of generating an aerosol by heating a cigarette. The
aerosol generating device may determine whether or not
a cigarette is inserted, by using at least one of a cigarette
insertion detecting sensor, a temperature detecting sensor configured to detect a temperature change of a heater, or a position detecting sensor configured to detect a
position change of a door, without receiving an additional
input from a user, and based on whether or not the cig-
arette is inserted, may control the operation of the heater.

FIG. 5

EP 4 298 936 A2

## Description

TECHNICAL FIELD

The present disclosure relates to a method and a device for generating an aerosol while having a function of detecting insertion of a cigarette.

BACKGROUND ART

Recently, demand has increased for alternative methods capable of overcoming defects of a general cigarette. For example, there has been increasing demand for a method of generating an aerosol by heating an aerosol generating material in a cigarette, rather than a method of generating an aerosol by burning a cigarette.

When an electronic cigarette device, which includes a heater for heating a cigarette based on electricity, is used, the heater may sometimes operate in a state in which the cigarette is not inserted into the electronic cigarette device, which may cause unnecessary power consumption.

Also, when the cigarette is inserted into the electronic cigarette device, the heater may operate only after the electronic cigarette device receives an additional input from the user, and thus, a time taken for the cigarette to be heated to a targeted temperature may be increased.

Therefore, research on heated aerosol generating devices has been actively conducted.

DESCRIPTION OF EMBODIMENTS

TECHNICAL PROBLEM

Provided are a method and a device for generating an aerosol while having a function of detecting insertion of a cigarette. An aerosol generating device may determine whether or not a cigarette is inserted, by using at least one of a cigarette insertion detecting sensor, a temperature detecting sensor configured to detect a temperature change of a heater, or a position detecting sensor configured to detect a position change of a door, without receiving an additional input from a user. An aerosol generating device may control an operation of a heater based on whether or not a cigarette is inserted.

Technical objectives of embodiments are not limited to the described technical objectives and other technical objectives may be derived from the embodiments to be described hereinafter.

SOLUTION TO PROBLEM

An aerosol generating device for generating an aerosol by heating a cigarette and a method of generating an aerosol by heating a cigarette are provided.

The aerosol generating device may determine whether or not a cigarette is inserted, by using at least one of a cigarette insertion detecting sensor, a temperature detecting sensor configured to detect a temperature change of a heater, or a position detecting sensor configured to detect a position change of a door, without receiving an additional input from a user. Also, the aerosol generating device may control an operation of the heater based on whether or not the cigarette is inserted.

ADVANTAGEOUS EFFECTS OF DISCLOSURE

In contrast to an aerosol generating device according to the conventional art, a user is provided with an aerosol generating device configured to determine whether or not a cigarette is inserted, without receiving an additional input from the user, and to control an operation of the heater based on whether or not the cigarette is inserted.

BRIEF DESCRIPTION OF DRAWINGS

FIGS. 1 through 3 are diagrams showing examples in which a cigarette is inserted into an aerosol generating device.
FIG. 4 shows an example of a cigarette.
FIG. 5 is a perspective view of an aerosol generating device according to an embodiment.
FIG. 6 is a lateral cross-sectional view of some components of the aerosol generating device according to the embodiment illustrated in FIG. 5.
FIG. 7 is a flowchart for describing a method of detecting insertion of a cigarette, the method being performed by a cigarette insertion detecting sensor, according to an embodiment.
FIG. 8 is a lateral cross-sectional view of a cigarette insertion detecting sensor located inside an accommodation passage, according to an embodiment.
FIG. 9 is a flowchart for describing a method of determining whether or not a cigarette is accommodated, based on a temperature change of a heater, according to an embodiment.
FIG. 10 is a flowchart for describing a method of determining whether or not a cigarette is inserted, based on a position change of a door, according to an embodiment.
FIG. 11 is a block diagram of hardware components of an aerosol generating device.

BEST MODE

According to an aspect of the present disclosure, an aerosol generating device includes: an accommodation passage configured to accommodate a cigarette; a heater configured to heat the cigarette accommodated in the accommodation passage; a cigarette insertion detecting sensor located inside the accommodation passage; and a controller, wherein the controller is configured to detect whether or not the cigarette is inserted into the accommodation passage by using the cigarette insertion detecting sensor.

According to another aspect of the present disclosure, a method, performed by an aerosol generating device, of generating an aerosol by heating a cigarette, includes: receiving, from a cigarette insertion detecting sensor located inside an accommodation passage, a signal of detecting whether or not the cigarette is inserted into the accommodation passage; determining, based on the received signal, whether or not the cigarette is inserted into the accommodation passage; and controlling, based on whether or not the cigarette is inserted into the accommodation passage, an operation of a heater.

According to another aspect of the present disclosure, an aerosol generating device includes: an accommodation passage configured to accommodate a cigarette; a heater configured to heat the cigarette accommodated in the accommodation passage; a temperature detecting sensor configured to detect a temperature change of the heater; and a controller, wherein the controller is configured to receive, from the temperature detecting sensor, a signal of detecting the temperature change of the heater and determine, based on the received signal, whether or not the cigarette is accommodated in the accommodation passage.

According to another aspect of the present disclosure, a method, performed by an aerosol generating device, of generating an aerosol by heating a cigarette, includes: receiving, from a temperature detecting sensor configured to detect a temperature change of a heater, a signal of detecting the temperature change of the heater; determining whether a value of the temperature change of the heater, the value being derived from the received signal, corresponds to a value of a reference temperature change corresponding to a case in which the cigarette is accommodated in an accommodation passage, by comparing the value of the temperature change of the heater with the value of the reference temperature change; and determining, based on a result of the determination, whether or not the cigarette is accommodated in the accommodation passage.

According to another aspect of the present disclosure, a method, performed by an aerosol generating device, of generating an aerosol by heating a cigarette, includes: receiving, from a position detecting sensor configured to detect a position change of a door included at an upper surface of a cover and exposing an accommodation passage configured to accommodate the cigarette to the outside, a signal of detecting the position change of the door; and determining, based on the received signal, whether or not the cigarette is inserted into the accommodation passage.

According to another aspect of the present disclosure, an aerosol generating device includes: a cover; an accommodation passage configured to accommodate a cigarette inserted via the cover; a heater configured to heat the cigarette accommodated in the accommodation passage; a temperature detecting sensor configured to detect a temperature change of the heater; and a controller, wherein the cover includes: a door included at an upper surface of the cover and exposing the accommodation passage to the outside; and a position detecting sensor configured to detect a position change of the door, and the controller is configured to receive, from the temperature detecting sensor, a signal of detecting the temperature change of the heater, and from the position detecting sensor, a signal of detecting the position change of the door, and determine, based on the signal received from the temperature detecting sensor and the signal received from the position detecting sensor, whether or not the cigarette is inserted into the accommodation passage.

According to another aspect of the present disclosure, a method, performed by an aerosol generating device, of generating an aerosol by heating a cigarette, includes: receiving, from a temperature detecting sensor configured to detect a temperature change of a heater, a signal of detecting the temperature change of the heater; receiving, from a position detecting sensor configured to detect a position change of a door included at an upper surface of a cover and exposing an accommodation passage configured to accommodate the cigarette to the outside, a signal of detecting the position change of the door; and determining, based on the signal received from the temperature detecting sensor and the signal received from the position detecting sensor, whether or not the cigarette is accommodated in the accommodation passage.

MODE OF DISCLOSURE

With respect to the terms in the various embodiments of the present disclosure, the general terms which are currently and widely used are selected in consideration of functions of structural elements in the various embodiments of the present disclosure. However, meanings of the terms may be changed according to intention, a judicial precedent, appearance of a new technology, and the like. In addition, in certain cases, a term which is not commonly used may be selected. In such a case, the meaning of the term will be described in detail at the corresponding part in the description of the present disclosure. Therefore, the terms used in the various embodiments of the present disclosure should be defined based on the meanings of the terms and the descriptions provided herein.

In addition, unless explicitly described to the contrary, the word "comprise" and variations such as "comprises" or "comprising" will be understood to imply the inclusion of stated elements but not the exclusion of any other elements. Also, the terms, such as "unit" or "module," described in the specification, denote a unit that processes at least one function or operation and that may be embodied in a hardware manner, a software manner, or a combination of the hardware manner and the software manner.

Hereinafter, exemplary embodiments of the present disclosure will be described in detail with reference to the accompanying drawings. The disclosure may, however, be embodied in many different forms and should not be construed as limited to the embodiments set forth herein.

Hereinafter, embodiments of the present disclosure will be described in detail with reference to the drawings.

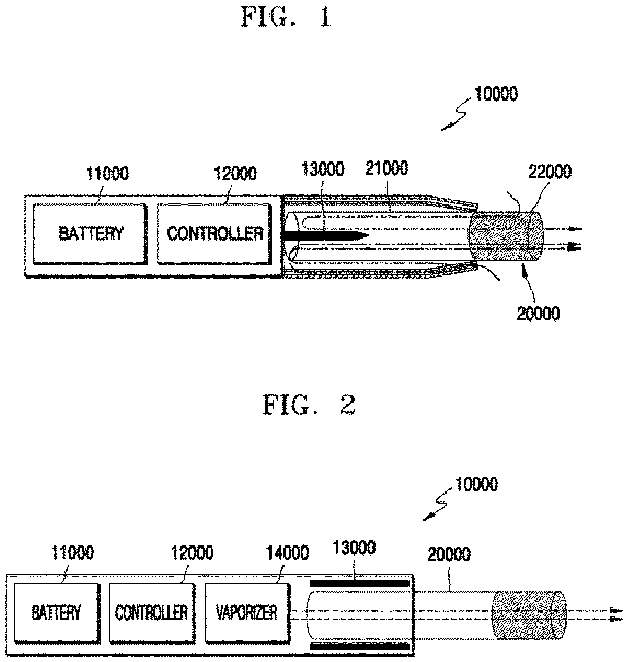

FIGS. 1 through 3 are diagrams showing examples in which a cigarette 20000 is inserted into an aerosol generating device 10000.

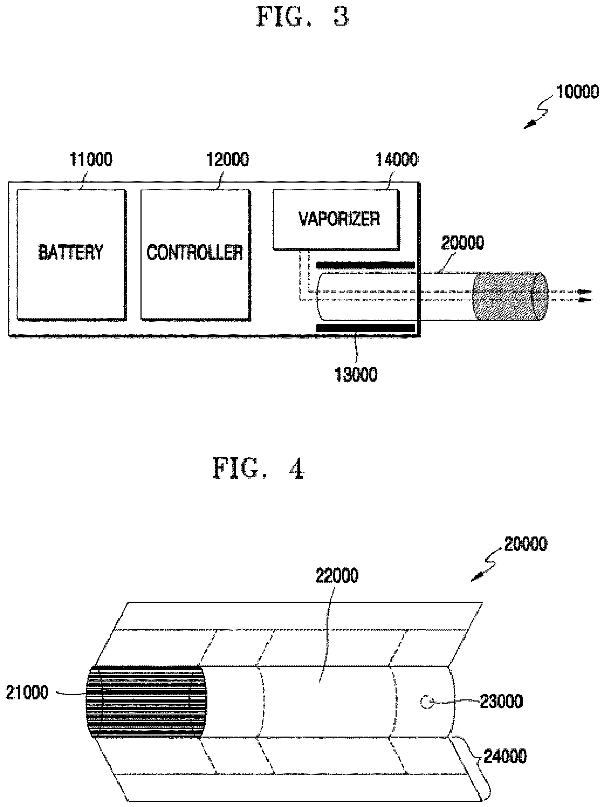

Referring to FIG. 1, the aerosol generating device 10000 may include a battery 11000, a controller 12000, and a heater 13000. Referring to FIGS. 2 and 3, the aerosol generating device 10000 may further include a vaporizer 14000. Also, the cigarette 20000 may be inserted into an inner space of the aerosol generating device 10000.

FIGS. 1 through 3 illustrate only components of the aerosol generating device 10000, which are related to the present embodiment. Thus, one of ordinary skill in the art may understand that the aerosol generating device 10000 may further include other general-purpose components, in addition to the components illustrated in FIGS. 1 through 3.

Also, FIGS. 2 and 3 illustrate that the aerosol generating device 10000 includes the heater 13000. However, according to necessity, the heater 13000 may be omitted.

FIG. 1 illustrates that the battery 11000, the controller 12000, and the heater 130000 are arranged in series. Also, FIG. 2 illustrates that the battery 11000, the controller 12000, the vaporizer 14000, and the heater 13000 are arranged in series. Also, FIG. 3 illustrates that the vaporizer 14000 and the heater 13000 are arranged in parallel. However, the internal structure of the aerosol generating device 10000 is not limited to the structures illustrated in FIGS. 1 through 3. In other words, according to the design of the aerosol generating device 10000, the battery 11000, the controller 12000, the heater 13000, and the vaporizer 14000 may be differently arranged.

When the cigarette 20000 is inserted into the aerosol generating device 10000, the aerosol generating device 10000 may operate the heater 13000 and/or the vaporizer 14000 to generate an aerosol from the cigarette 20000 and/or the vaporizer 14000. The aerosol generated via the heater 13000 and/or the vaporizer 14000 may be delivered to a user by passing through the cigarette 20000.

According to necessity, even when the cigarette 20000 is not inserted into the aerosol generating device 10000, the aerosol generating device 10000 may heat the heater 13000.

The battery 11000 may supply power to be used for the aerosol generating device 10000 to operate. For example, the battery 11000 may supply power to heat the heater 13000 or the vaporizer 14000 and may supply power for an operation of the controller 12000. Also, the battery 11000 may supply power for operations of a display, a sensor, a motor, etc. mounted in the aerosol generating device 10000.

The controller 12000 may generally control operations of the aerosol generating device 10000. In detail, the controller 12000 may control not only operations of the battery 11000, the heater 13000, and the vaporizer 14000, but also operations of other components included in the aerosol generating device 10000. Also, the controller 12000 may check a state of each of the components of the aerosol generating device 10000 to determine whether or not the aerosol generating device 10000 is able to operate.

The controller 12000 may include at least one processor. The processor may be realized as an array of a plurality of logic gates or may be realized as a combination of a general-purpose microprocessor and a memory storing a program executable in the microprocessor. It will be understood by one of ordinary skill in the art that the disclosure may be realized as other forms of hardware.

The heater 13000 may be heated by the power supplied from the battery 11000. For example, when the cigarette 20000 is inserted into the aerosol generating device 10000, the heater 13000 may be located inside the cigarette 20000. Thus, the heated heater 13000 may increase a temperature of an aerosol generating material in the cigarette 20000.

The heater 13000 may include an electro-resistive heater. For example, the heater 13000 may include an electrically conductive track and the heater 13000 may be heated when currents flow through the electrically conductive track. However, the heater 13000 is not limited to the example described above and may include all heaters which may be heated to a desired temperature. Here, the desired temperature may be pre-set in the aerosol generating device 10000 or may be set as a temperature desired by a user.

As another example, the heater 13000 may include an induction heating heater. In detail, the heater 13000 may include an electrically conductive coil for heating a cigarette in an induction heating method, and the cigarette may include a susceptor which may be heated by the induction heating heater.

For example, the heater 13000 may include a tube-type heating element, a plate-type heating element, a needle-type heating element, or a rod-type heating element, and may heat the inside or the outside of the cigarette 20000, according to the shape of the heating element.

Also, the aerosol generating device 10000 may include a plurality of heaters 13000. Here, the plurality of heaters 13000 may be inserted in the cigarette 20000 or may be arranged outside the cigarette 20000. Also, one or more of the plurality of heaters 13000 may be inserted in the cigarette 20000 and the others may be arranged outside the cigarette 20000. In addition, the shape of the heater 13000 is not limited to the shapes illustrated in FIGS. 1 through 3 and may include various shapes.

The vaporizer 14000 may generate an aerosol by heating a liquid composition and the generated aerosol may pass through the cigarette 20000 to be delivered to a user. In other words, the aerosol generated via the vaporizer 14000 may move along an air current passage of the aerosol generating device 10000 and the air current passage may be configured such that the aerosol generated via the vaporizer 14000 passes through the cigarette 20000 to be delivered to the user.

For example, the vaporizer 14000 may include a liquid storage, a liquid transmitting device, and a heating element, but it is not limited thereto. For example, the liquid storage, the liquid transmitting device, and the heating element may be included in the aerosol generating device 10000 as independent modules.

The liquid storage may store a liquid composition. For example, the liquid composition may be a liquid including a tobacco-containing material having a volatile tobacco flavor component, or a liquid including a non-tobacco material. The liquid storage may be formed to be attached/detached to/from the vaporizer 14000 or may be formed integrally with the vaporizer 14000.

For example, the liquid composition may include water, a solvent, ethanol, plant extract, spices, flavorings, or a vitamin mixture. The spices may include menthol, peppermint, spearmint oil, and various fruit-flavored ingredients, but are not limited thereto. The flavorings may include ingredients capable of providing various flavors or tastes to a user. The vitamin mixture may include at least one of vitamin A, vitamin B, vitamin C, and vitamin E, but is not limited thereto. Also, the liquid composition may include an aerosol forming substance, such as glycerin and propylene glycol.

The liquid transmitting device may transmit the liquid composition in the liquid storage to the heating element. For example, the liquid transmitting device may include wicks, such as cotton fiber, ceramic fiber, glass fiber, and porous ceramics, but it is not limited thereto.

The heating element may be an element for heating the liquid composition transmitted by the liquid transmitting device. For example, the heating element may include a metal heat wire, a metal hot plate, a ceramic heater, etc., but it is not limited thereto. Also, the heating element may include a conductive filament, such as a nichrome wire, and may be coiled at the liquid transmitting device. The heating element may be heated via a supply of power and may provide heat to a liquid composition contacting the heating element so that the liquid composition is heated. As a result, an aerosol may be generated.

For example, the vaporizer 14000 may be referred to as a cartomizer or an atomizer, but it is not limited thereto.

The aerosol generating device 10000 may further include general-purpose components in addition to the battery 11000, the controller 12000, the heater 13000, and the vaporizer 14000. For example, the aerosol generating device 10000 may include a display capable of outputting visual information and/or a motor for outputting haptic information. Also, the aerosol generating device 10000 may include at least one sensor (a puff detecting sensor, a temperature detecting sensor, a cigarette insertion detecting sensor, etc.). Also, the aerosol generating device 10000 may be formed as a structure where, even when the cigarette 20000 is inserted into the aerosol generating device 10000, external air may be introduced or internal air may be discharged.

Although not illustrated in FIGS. 1 through 3, the aerosol generating device 10000 may be included in a system, together with an additional cradle. For example, the cradle may be used to charge the battery 11000 of the aerosol generating device 10000. Alternatively, the heater 13000 may be heated when the cradle and the aerosol generating device 10000 are attached to each other.

The cigarette 20000 may be similar as a general combustive cigarette. For example, the cigarette 20000 may be divided into a first portion including an aerosol generating material and a second portion including a filter, etc. Alternatively, the second portion of the cigarette 20000 may also include an aerosol generating material. For example, a granular or capsular aerosol generating material may be inserted into the second portion.

The entire first portion may be inserted into the aerosol generating device 10000 and the second portion may be exposed to the outside. Alternatively, a portion of the first portion may be inserted into the aerosol generating device 10000. Alternatively, the entire first portion and a portion of the second portion may be inserted into the aerosol generating device 10000. The user may inhale the aerosol while biting the second portion with a mouth. Here, the aerosol may be generated as the external air passes through the first portion, and the generated aerosol may pass through the second portion to be delivered to the mouth of the user.

For example, the external air may flow into at least one air passage formed in the aerosol generating device 10000. For example, the opening and closing and/or a size of the air passage formed in the aerosol generating device 10000 may be adjusted by the user. Accordingly, an amount of smoke and a smoking impression may be adjusted by the user. As another example, the external air may flow into the cigarette 20000 through at least one hole formed in a surface of the cigarette 20000.

Hereinafter, an example of the cigarette 20000 will be described with reference to FIG. 4.

FIG. 4 is shows an example of the cigarette 20000.

Referring to FIG. 4, the cigarette 20000 may include a tobacco rod 21000 and a filter rod 22000. The first portion described above with reference to FIGS. 1 through 3 may include the tobacco rod 21000, and the second portion may include the filter rod 22000.

FIG. 4 illustrates that the filter rod 22000 includes a single segment. However, the filter rod 22000 is not limited thereto. In other words, the filter rod 22000 may include a plurality of segments. For example, the filter rod 22000 may include a first segment configured to cool an aerosol and a second segment configured to filter a certain component included in the aerosol. Also, according to necessity, the filter rod 22000 may further include at least one segment configured to perform other functions.

The cigarette 2000 may be packaged via at least one wrapper 24000. The wrapper 24000 may have at least one hole through which external air may be introduced or internal air may be discharged. For example, the cigarette 20000 may be packaged via the at least one wrapper 24000. As another example, the cigarette 20000 may be doubly packaged via at least two wrappers 24000. For example, the tobacco rod 21000 may be packaged via a first wrapper, and the filter rod 22000 may be packaged via a second wrapper. Also, the tobacco rod 21000 and the filter rod 22000, each of which is packaged via separate wrappers, may be coupled to each other, and the entire cigarette 20000 may be packaged via a third wrapper. When each of the tobacco rod 21000 and the filter rod 22000 includes a plurality of segments, each segment may be packaged via a separate wrapper. Also, the entire cigarette 20000 including the plurality of segments, each of which is packaged via the separate wrappers and which are coupled to each other, may be repackaged via another wrapper.

The tobacco rod 21000 may include an aerosol generating material. For example, the aerosol generating material may include at least one of glycerin, propylene glycol, ethylene glycol, dipropylene glycol, diethylene glycol, triethylene glycol, tetraethylene glycol, or oleyl alcohol, but it is not limited thereto. Also, the tobacco rod 21000 may include other additives, such as flavors, a wetting agent, and/or organic acid. Also, the tobacco rod 21000 may include a flavored liquid, such as menthol or a moisturizer, which is injected to the tobacco rod 21000.

The tobacco rod 21000 may be manufactured in various forms. For example, the tobacco rod 21000 may be formed as a sheet or a strand. Also, the tobacco rod 21000 may be formed as a pipe tobacco, which is formed of tiny bits cut from a tobacco sheet. Also, the tobacco rod 21000 may be surrounded by a heat conductive material. For example, the heat conductive material may include a metal foil, such as an aluminum foil, but it is not limited thereto. For example, the heat conductive material surrounding the tobacco rod 21000 may uniformly distribute heat transmitted to the tobacco rod 21000, and thus, the heat conductivity applied to the tobacco rod may be increased and taste of the tobacco may be improved. Also, the heat conductive material surrounding the tobacco rod 21000 may function as a susceptor heated by the induction heating heater. Here, although not illustrated in the drawings, the tobacco rod 21000 may further include an additional susceptor, in addition to the heat conductive material surrounding the tobacco rod 21000.

The filter rod 22000 may include a cellulose acetate filter. Shapes of the filter rod 22000 are not limited. For example, the filter rod 22000 may include a cylinder-type rod or a tube-type rod having a hollow inside. Also, the filter rod 22000 may include a recess-type rod. When the filter rod 22000 includes a plurality of segments, at least one of the plurality of segments may have a different shape.

The filter rod 22000 may be formed to generate flavors. For example, a flavoring liquid may be injected onto the filter rod 22000, or an additional fiber coated with a flavoring liquid may be inserted into the filter rod 22000.

Also, the filter rod 22000 may include at least one capsule 23000. Here, the capsule 23000 may generate a flavor or an aerosol. For example, the capsule 23000 may include a film wrapping a content liquid including a spice. The capsule 23000 may have a spherical or a cylindrical shape, but it is not limited thereto.

When the filter rod 22000 includes a segment configured to cool the aerosol, the cooling segment may include a polymer material or a biodegradable polymer material. For example, the cooling segment may include only a pure polylactic acid, but it is not limited thereto. Alternatively, the cooling segment may include a cellulose acetate filter having a plurality of holes. However, the cooling segment is not limited to the examples described above and may include all cooling segments capable of cooling an aerosol.

Although not illustrated in FIG. 4, the cigarette 20000 according to an embodiment may further include a front-end filter. The front-end filter may be located on a side of the tobacco rod 21000, the side facing the filter rod 22000. The front-end filter may prevent the tobacco rod 21000 from being detached outwards and prevent a liquefied aerosol from flowing into the aerosol generating device 10000 (FIGS. 1 through 3) from the tobacco rod 21000, during smoking.

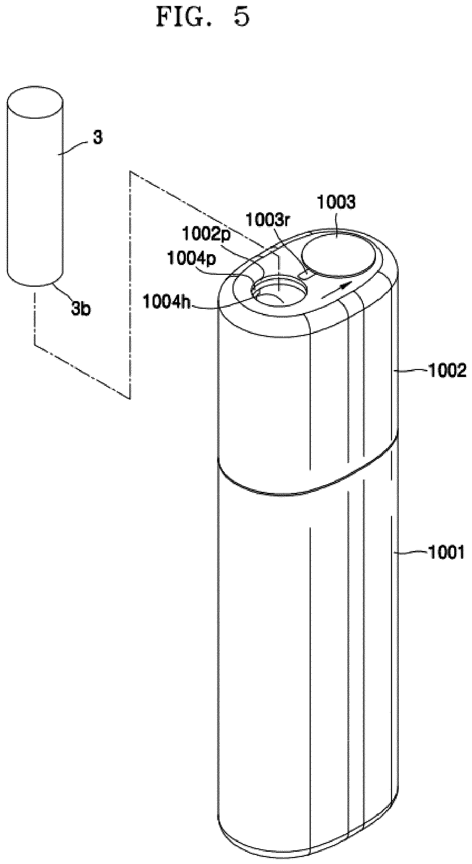

FIG. 5 is a perspective view of an aerosol generating device 1000 according to an embodiment.

An aerosol generating source support assembly and the aerosol generating device 1000 including the same may include a case 1001 and a cover 1002, according to the embodiment illustrated in FIG. 5. The cover 1002 may be coupled to an end of the case 1001 so that the case 1001 and the cover 1002 may form an exterior of the aerosol generating device 1000.

The case 1001 may form a portion of the exterior of the aerosol generating device 1000, and may accommodate various components to protect the components.

The cover 1002 and the case 1001 may be formed of a plastic material having a low thermal conductivity, or a metal material having a surface coated with a heat blocking material. The cover 1002 and the case 1001 may be formed by using, for example, an injection molding method, a three-dimensional (3D) printing method, or a method in which small components manufactured by using an injection molding method are assembled.

A retaining device may be formed between the cover 1002 and the case 1001 to retain the coupling between the cover 1002 and the case 1001. The retaining device may include, for example, a projection and a groove. The cover 1002 and the case 1001 may be kept being coupled to each other by the projection being inserted into the groove, and the projection may be separated from the groove via a movement of the projection based on a manipulation button which may be pressed by a user.

Also, the retaining device may include, for example, a magnet and a metal member attached to the magnet. When the retaining device includes a magnet, the magnet may be formed at any one of the cover 1002 and the case 1001, and a metal member attached to the magnet may be formed at the other. Alternatively, the magnet may be formed at both of the cover 1002 and the case 1001.

The cover 1002 is not an essential component of the aerosol generating device 1000 according to the embodiment illustrated in FIG. 5, and thus, when necessary, the cover 1002 may not be formed.

An external hole 1002p through which a cigarette 3 may be inserted may be formed in an upper surface of the cover 1002 coupled to the case 1001. Also, a rail 1003r may be formed on a location of the upper surface of the cover 1002, the location being adjacent to the external hole 1002p. A door 1003 slidingly movable along the upper surface of the cover 1002 may be formed at the rail 1003r. The door 1003 may move slidingly and linearly along the rail 1003r.

The door 1003 may move along the rail 1003 in the arrow direction of FIG. 5 and may expose the external hole 1002p and an insertion hole 1004p, through which the cigarette 3 passes through the cover 1002 and is inserted into the case 1001. The external hole 1002p of the cover 1002 may expose the insertion hole 1004p of an accommodation passage 1004h in which the cigarette 3 is accommodated.

When the external hole 1002p is exposed through the door 1003, a user may insert an end 3b of the cigarette 3 into the external hole 1002p and the insertion hole 1004p and mount the cigarette 3 in the accommodation passage 1004h formed inside the cover 1002.

According to an embodiment, the door 1003 may be formed to move linearly with respect to the cover 1002. However, embodiments are not limited to the structure in which the door 1003 is coupled to the cover 1002. For example, the door 1003 may be rotatably formed at the cover 1002 via a hinge assembly. When the hinge assembly is used, the door 1003 may rotate to a side surface of the external hole 1002p in a direction in which the upper surface of the cover 1002 extends, or may rotate in a direction away from the upper surface of the cover 1002.

The rail 1003r may have a shape of a concave groove. However, embodiments are not limited to this shape of the rail 1003r. For example, the rail 1003r may have a convex shape and may extend in a curved form rather than a linear form.

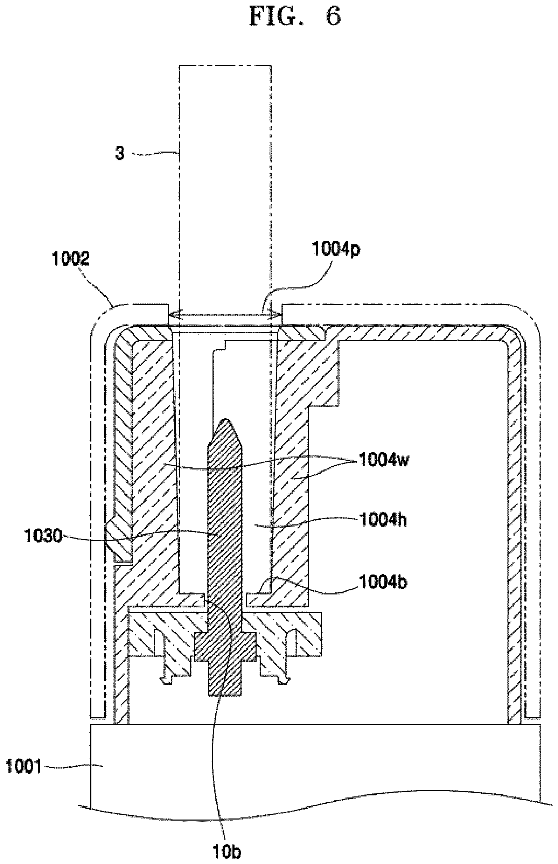

FIG. 6 is a lateral cross-sectional view of some components of the aerosol generating device 1000 according to the embodiment illustrated in FIG. 5.

Referring to FIG. 6, the cigarette 3 may be inserted into the insertion hole 1004p from the outside and accommodated in the accommodation passage 1004h. An end of the heater 1030 may pass through a heater insertion hole 10b to be located in the accommodation passage 1004h. Thus, when the cigarette 3 is accommodated in the accommodation passage 1004h, the end of the heater 1030 may be inserted into the cigarette 3.

An accommodation portion configured to accommodate the cigarette 3 may include: a side wall 1004w forming the accommodation passage 1004h configured to accommodate the cigarette 3; the insertion hole 1004p at an end of the accommodation passage 1004h, the insertion hole 1004p being open to the outside so that the cigarette 3 may be inserted; and a bottom wall 1004b closing the other end of the accommodation passage 1004h and having a heater hole 10b through which the end of the heater 1030 passes.

According to the embodiment illustrated in FIG. 6, the heater may be inserted into the cigarette. However, embodiments are not limited to this structure of the heater. For example, the heater may be formed as a cylinder-shaped film-type heater configured to surround at least a portion of an external surface of the cigarette. When the cylindrical film-type heater is used, the cylindrical film-type heater may be formed at the side wall 1004w forming the accommodation passage 1004h.

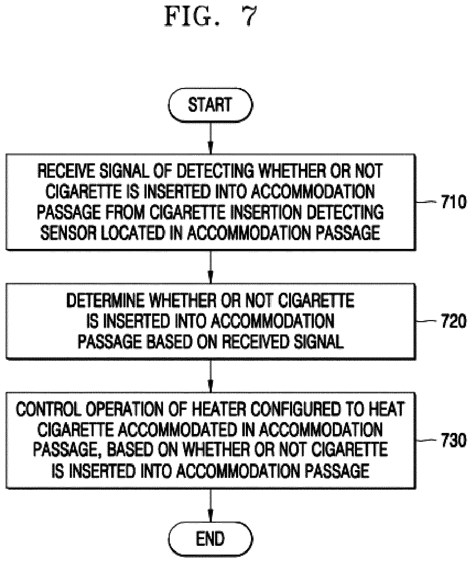

FIG. 7 is a flowchart for describing a method of detecting insertion of a cigarette, the method being performed by a cigarette insertion detecting sensor, according to an embodiment.

Referring to FIG. 7, in operation 710, an aerosol generating device may receive a signal of detecting whether or not a cigarette is inserted into an accommodation passage, from a cigarette insertion detecting sensor located inside the accommodation passage.

The cigarette insertion detecting sensor may detect that the cigarette is inserted into the accommodation passage, when the cigarette, after being inserted into an insertion hole of a cover, moves along the accommodation passage in a direction toward a bottom wall of the accommodation passage, that is, moves downwardly. After the cigarette insertion detecting sensor detects the insertion of the cigarette, the cigarette insertion detecting sensor may transmit an insertion detecting signal to a controller of the aerosol generating device.

The cigarette insertion detecting sensor may be located inside the accommodation passage. The cigarette insertion detecting sensor may be mounted at a side wall or the bottom wall of the accommodation passage. However, locations at which the cigarette insertion detecting sensor is mounted are not limited thereto.

In operation 720, the aerosol generating device may determine whether or not the cigarette is inserted into the accommodation passage, based on the received signal.

According to an embodiment, the cigarette insertion detecting sensor may include at least one of a film sensor, a pressure sensor, an optical sensor, or an infrared sensor.

The film sensor may be configured to detect an aerosol generating material included in the cigarette. The pressure sensor may be configured to detect pressure that is applied to the side wall or the bottom wall of the accommodation passage, when the cigarette, after being inserted into the insertion hole of the cover, downwardly moves along the accommodation passage. The optical sensor and the infrared sensor may detect the cigarette's blocking of light and infrared rays in the process in which the cigarette moves in the downward direction along the accommodation passage after being inserted into the insertion hole of the cover.

The aerosol generating device may determine whether or not the cigarette is inserted into the accommodation passage, by receiving, from the cigarette insertion detecting sensor, the signal with respect to whether or not the cigarette is inserted.

In operation 730, the aerosol generating device may control an operation of a heater, based on whether or not the cigarette is inserted in to the accommodation passage.

According to an embodiment, the aerosol generating device may initiate the operation of the heater, after receiving, from the cigarette insertion detecting sensor, the signal that the cigarette is inserted. Here, the aerosol generating device may initiate the operation of the heater by initiating an operation of a power supply device. For example, after the aerosol generating device receives the signal that the cigarette is inserted from the cigarette insertion detecting sensor, the aerosol generating device may supply power to the heater to allow the heater to enter into a heating mode or a pre-heating mode.

In the heating mode, a temperature of the heater may increase to a target temperature so that the aerosol generating material of the cigarette may be heated to generate an aerosol, and in the pre-heating mode, the temperature of the heater may be kept to be lower than the target temperature. However, operations of the heating mode and the pre-heating mode are not limited thereto.

Because the aerosol generating device may initiate the operation of the heater based on the signal received from the cigarette insertion detecting sensor, the aerosol generating device may, without receiving an additional input from a user, set the heater in the heating mode or the pre-heating mode. Accordingly, time taken for the heater to reach the target temperature may be reduced. When the heater is set in the pre-heating mode, the heater may operate in the heating mode when an additional input is received from the user.

Also, the aerosol generating device may stop the operation of the heater after receiving, from the cigarette insertion detecting sensor, a signal that the cigarette is removed from the cigarette. Here, the aerosol generating device may block the power supplied from the power supply device to the heater. For example, the aerosol generating device may block the power supplied to the heater, after receiving, from the cigarette insertion detecting sensor, the signal that the cigarette is removed.

Because the aerosol generating device may stop the operation of the heater based on the signal received from the cigarette insertion detecting sensor, the aerosol generating device may stop the operation of the heater by removing the cigarette inserted into the accommodation passage, without receiving an additional input from the user.

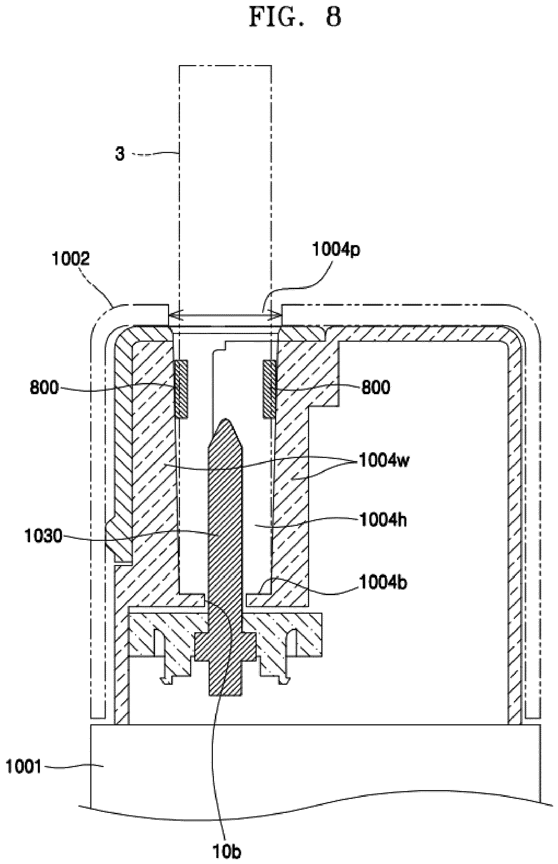

FIG. 8 is a lateral cross-sectional view of a cigarette insertion detecting sensor 800 located inside the accommodation passage 1004h, according to an embodiment.

Referring to FIG. 8, the cigarette 3 may pass through the cover 1002 to be inserted into the insertion hole 1004p and then may be accommodated in the accommodation passage 1004h.

The cigarette insertion detecting sensor 800 may be located inside the accommodation passage 1004h. The cigarette insertion detecting sensor 800 may be mounted at the side wall 1004w, the bottom wall 1004b, etc. forming the accommodation passage 1004h. However, locations of the cigarette insertion detecting sensor 800 are not limited thereto.

The cigarette insertion detecting sensor 800 according to an embodiment may include at least one of a film sensor, a pressure sensor, an optical sensor, or an infrared sensor.

The film sensor may be configured to detect only particular materials and may be configured to detect an aerosol generating material included in the cigarette 3. In a process in which the cigarette 3 is inserted into the insertion hole 1004 and downwardly moves along the accommodation passage 1004h, the film sensor may transmit, to a controller, a signal that the cigarette 3 is inserted into the accommodation passage 1004h, by detecting an aerosol generating material immersed in cigarette 3 or a material with which a surface of the cigarette 3 is coated.

When the cigarette 3 including only materials that may not be detected by the film sensor is inserted into the accommodation passage 1004h, the film sensor may not transmit the signal that the cigarette 3 is inserted to the controller, even when the cigarette 3 is inserted. That is, an operation of the heater 1030 may be initiated, only when the cigarette 3 is inserted into the accommodation passage 1004h, the cigarette 3 including particular materials which may be detected by the film sensor.

In a process in which the cigarette 3 is inserted into the insertion hole 1004p and downwardly moves along the accommodation passage 1004h, the pressure sensor may detect pressure applied by the cigarette 3 to the side wall 1004w or the bottom wall 1004b of the accommodation passage 1004h. After the pressure sensor detects the pressure as the cigarette 3 is inserted into the accommodation passage 1004h, the pressure sensor may transmit a signal that the cigarette 3 is inserted into the accommodation passage 1004h to the controller.

In a process in which the cigarette 3 is inserted into the insertion hole 1004p and downwardly moves along the accommodation passage 1004h, the optical sensor may detect the blocking of light by the cigarette 3. The optical sensor may include a light-emission portion and a light-reception portion, wherein the light-emission portion and the light-reception portion may be mounted in the accommodation passage 1004h to face each other.

When the cigarette 3 is inserted into the accommodation passage 1004h, light emitted through the light-emission portion may not be received by the light-reception portion. In this case, the optical sensor may transmit a signal that the cigarette 3 is inserted into the accommodation passage 1004 to the controller.

In a process in which the cigarette 3 is inserted into the insertion hole 1004p and downwardly moves along the accommodation passage 1004h, the infrared sensor may detect the blocking of infrared rays by the cigarette 3. An operation of the infrared sensor is the same as the operation of the optical sensor, and thus, its description will be omitted for convenience.

The aerosol generating device 1000 may receive a signal of detecting whether or not the cigarette 3 is inserted into the accommodation passage 1004h, from the cigarette insertion detecting sensor 800, and may control an operation of a heater 1030 based on the received signal.

According to the aerosol generating device 1000, when a user inserts the cigarette 3 into the aerosol generating device 1000, the cigarette insertion detecting sensor 800 may detect the insertion of the cigarette 3 and transmit the cigarette insertion detecting signal. When the controller receives, from the cigarette insertion detecting sensor 800, the cigarette insertion detecting signal that detects that the cigarette 3 is inserted, the controller may set the heater 1030 in a pre-heating mode to heat an aerosol generating material of the cigarette 3. When the controller does not receive, from the cigarette insertion detecting sensor 800, the cigarette insertion detecting signal that detects that the cigarette 3 is inserted, the controller may not set the heater 1030 in the pre-heating mode.

Also, when the controller does not receive the cigarette insertion detecting signal that detects that the cigarette 3 is inserted, from the cigarette insertion sensing sensor 800, because the user has withdrawn the cigarette 3 from the aerosol generating device while using the cigarette 3 by inhaling an end portion of the cigarette 3 which is inserted into the aerosol generating device or after finishing using the cigarette 3, the controller may block a power supply to the heater 1003 to stop the operation of the heater 1030, thereby preventing the aerosol generating device 1000 from unnecessarily operating.

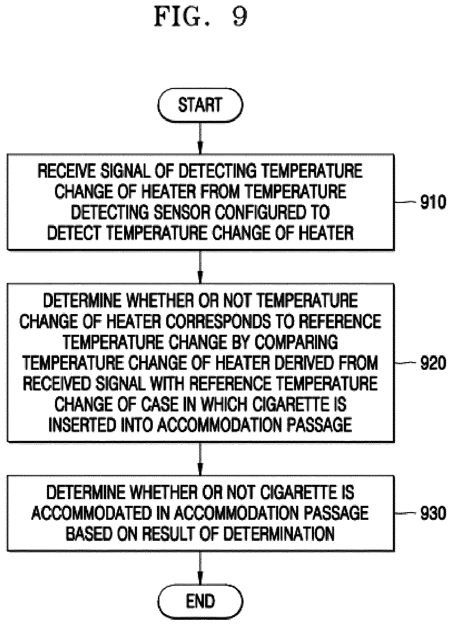

FIG. 9 is a flowchart for describing a method of determining whether or not a cigarette is accommodated, based on a temperature change of a heater, according to an embodiment.

Referring to FIG. 9, in operation 910, an aerosol generating device may receive, from a temperature detecting sensor configured to detect a temperature change of the heater, a signal of detecting the temperature change of the heater.

An additional temperature detecting sensor may be provided in an accommodation passage of the aerosol generating device. Alternatively, the temperature detecting sensor may not be included in the aerosol generating device and the heater may function as the temperature detecting sensor. Alternatively, the heater of the aerosol generating device may function as the temperature detecting sensor, and at the same time, an additional temperature detecting sensor may further be included in the accommodation passage of the aerosol generating device.

In order that the heater functions as the temperature detecting sensor, the heater may include at least one electrically conductive track for emitting heat and detecting temperature. Alternatively, the heater may include a first electrically conductive track for emitting heat and a second electrically conductive track for detecting temperature.

The temperature detecting sensor may transmit the signal of detecting the temperature change of the heater to a controller of the aerosol generating device.

In operation 920, the aerosol generating device may compare a value of the temperature change of the heater, the value being derived from the received signal, with a value of a reference temperature change corresponding to a case in which a cigarette is accommodated in the accommodation passage, to determine whether or not the value of the temperature change of the heater corresponds to the value of the reference temperature change.

The controller of the aerosol generating device may derive the value of the temperature change of the heater from the signal received from the temperature detecting sensor. The value of the temperature change of the heater may be a value of a temperature increase/decrease of the heater during a certain time period. A memory of the aerosol generating device may store data indicating the value of the reference temperature change of the case in which the cigarette is accommodated in the accommodation passage. The value of the reference temperature change may be data indicating a temperature change of or around the heater when power is supplied to the heater in a state in which the cigarette is accommodated in the accommodation passage. The value of the reference temperature change may be a value of a temperature increase/decrease of or around the heater during a certain time period.

The controller may compare the value of the temperature change of the heater, the value being derived from the signal received from the temperature detecting sensor, with the value of the reference temperature change stored in the memory.

When the value of the temperature change of the heater corresponds to the value of the reference temperature change, the controller may determine that the cigarette is accommodated in the accommodation passage. When the value of the temperature change of the heater does not correspond to the value of the reference temperature change, the controller may not determine that the cigarette is accommodated in the accommodation passage.

Here, that the value of the temperature change of the heater does not correspond to the value of the reference temperature change may include a case in which the value of the temperature change of the heater is greater than the value of the reference temperature change, that is, a case in which the value of the temperature change of the heater indicates a higher per-hour temperature change rate than the value of the reference temperature change. This aspect may be based on the fact that a temperature around the heater may be more quickly increased when the cigarette is not accommodated in the accommodation passage than when the cigarette is accommodated in the accommodation passage.

In operation 930, the aerosol generating device may determine whether or not the cigarette is accommodated in the accommodation passage based on a result of the determination of operation 920.

Also, the aerosol generating device may determine whether or not the cigarette is accommodated in the accommodation passage, and based thereon, may control an operation of the heater.

When the value of the temperature change of the heater corresponds to the value of the reference temperature change, the aerosol generating device may determine that the cigarette is accommodated in the accommodation passage and may initiate the operation of the heater. Here, the aerosol generating device may initiate the operation of the heater by initiating an operation of a power supply device.

The aerosol generating device may also compare the value of the temperature change of the heater with the value of the reference temperature change, even while the heater operates. Here, when the value of the temperature change of the heater corresponds to the value of the reference temperature change, the aerosol generating device may determine that the cigarette is accommodated in the accommodation passage and continue the operation of the heater.

Also, when the value of the temperature change of the heater does not correspond to the value of the reference temperature change, the aerosol generating device may determine that the cigarette is not accommodated in the accommodation passage and may stop the operation of the heater. Here, the aerosol generating device may block the power supplied from the power supply device to the heater.

When the cigarette is not accommodated in the accommodation passage, that is, when the cigarette is removed from the accommodation passage, the aerosol generating device may stop the operation of the heater, thereby preventing unnecessary power consumption and malfunction of the heater.

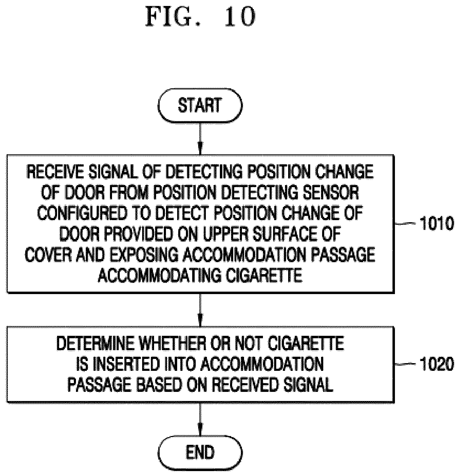

FIG. 10 is a flowchart for describing a method of determining whether or not a cigarette is inserted, based on a position change of a door, according to an embodiment.

Referring to FIG. 10, in operation 1010, an aerosol generating device may receive a signal of detecting the position change of the door, from a position detecting sensor configured to detect the position change of the door provided on an upper surface of a cover and capable of exposing an accommodation passage in which a cigarette is accommodated.

Referring to FIG. 5, the rail 1003r may be formed on the location of the upper surface of the cover 1002 coupled to the case 1001, the location being adjacent to the external hole 1002p. The door 1003 slidingly movable along the upper surface of the cover 1002 may be formed at the rail 1003r. The door 1003 may move linearly and slidingly along the rail 1003r. When the door 1003 moves along the rail 1003r in the arrow direction of FIG. 5, the accommodation passage 1004h in which the cigarette 3 is accommodated may be exposed to the outside. When the accommodation passage 1004h is exposed to the outside via the door 1003, a user may insert an end 3b of the cigarette 3 into the insertion hole 1004p so that the cigarette 3 may be accommodated in the accommodation passage 1004h.

According to an embodiment, the door 1003 may be formed to move linearly with respect to the cover 1002. However, structures in which the door 1003 is coupled to the cover 1002 are not limited thereto. For example, the door 1003 may be rotatably formed at the cover 1002 via a hinge assembly.

Hereinafter, a position of the door 1003 when the door 1003 closes the accommodation passage 1004h is referred to as a closing position and a position of the door 1003 when the door 1003 moves along the rail 1003r in the arrow direction of FIG. 5 to open the accommodation passage 1004h is referred to as an opening position.

Although not shown in FIG. 5, the cover 1002 may include a position detecting sensor configured to detect a position change of the door 1003. According to an embodiment, the position detecting sensor may detect whether the position of the door 1003 changes from the opening position to the closing position or from the closing position to the opening position. The position detecting sensor may transmit, after detecting the position change of the door 1003, a position change detecting signal to a controller of the aerosol generating device.

According to an embodiment, the position detecting sensor configured to detect the position change of the door 1003 may include a pressure sensor, a capacitance sensor, a resistance sensor, an orientation sensor, or a magnetic sensor, but it is not limited thereto.

In operation 1020, the aerosol generating device may determine whether or not the cigarette is inserted into the accommodation passage, based on the received signal.

When the aerosol generating device receives, from the position detecting sensor, the signal that the position of the door 1003 changes from the closing position to the opening position, the aerosol generating device may determine that the cigarette 3 is inserted into the accommodation passage 1004h and may initiate an operation of a heater. Here, the aerosol generating device may initiate the operation of the heater by initiating an operation of a power supply device.

The aerosol generating device may determine whether or not the cigarette is inserted based on the signal received from the position detecting sensor and may initiate the operation of the heater based on the determination, and thus, the aerosol generating device may set the heater in a heating mode or a pre-heating mode based on the position change of the door 1003 from the closing position to the opening position, without receiving an additional input from the user. Accordingly, time taken for the heater to reach the target temperature may be reduced. When the heater is set in the pre-heating mode, the heater may operate in the heating mode when an additional input is received from the user.

Also, when the aerosol generating device receives, from the position detecting sensor, the signal that the position of the door 1003 changes from the opening position to the closing position, the aerosol generating device may determine that the cigarette 3 is removed from the accommodation passage 1004h and may stop the operation of the heater. Here, the aerosol generating device may block the power supplied from the power supply device to the heater.

Because the aerosol generating device may stop the operation of the heater based on the signal received from the position detecting sensor, the aerosol generating device may stop the operation of the heater based on the position change of the door 1003 from the opening position to the closing position without receiving an additional input from the user.

According to an embodiment, an aerosol generating device may determine whether or not a cigarette is accommodated (or inserted) by combining the methods of FIGS. 9 and 10.

The aerosol generating device may receive the signal of detecting the temperature change of the heater from the temperature detecting sensor configured to detect the temperature change of the heater and the signal of detecting the position change of the door from the position detecting sensor configured to detect the position change of the door. Also, the aerosol generating device may determine whether or not the cigarette is accommodated in the accommodation passage based on the signal received from the temperature detecting sensor and the signal received from the position detecting sensor.

That is, the aerosol generating device may improve the reliability and accuracy of the determination with respect to the insertion of the cigarette, by determining whether or not the cigarette is accommodated (or inserted) into the accommodation passage based on both of the temperature change of the heater and the position change of the door.

The aerosol generating device may control an operation of the heater, based on whether or not the cigarette is accommodated in (or inserted into) the accommodation passage.

For example, when the aerosol generating device determines that a value of the temperature change of the heater, the value being obtained from the temperature detecting sensor, corresponds to a value of a reference temperature change, and receives the signal that the position of the door changes from the closing position to the opening position from the position detecting sensor, the aerosol generating device may determine that the cigarette is accommodated in (or inserted into) the accommodation passage.

Also, when the aerosol generating device determines that the value of the temperature change of the heater, the value being obtained from the temperature detecting sensor, does not correspond to the value of the reference temperature change, and receives the signal that the position of the door changes from the opening position to the closing position from the position detecting sensor, the aerosol generating device may determine that the cigarette is not accommodated in (or inserted into) the accommodation passage or is removed from the accommodation passage.

When the aerosol generating device determines that the cigarette is accommodated in (or inserted into) the accommodation passage, the aerosol generating device may initiate (or continue) the operation of the heater. When the aerosol generating device determines that the cigarette is not accommodated in (or inserted into) the accommodation passage, the aerosol generating device may stop the operation of the heater.

The aerosol generating device may determine the accommodation (or the insertion) of the cigarette based on the signals received from the temperature detecting sensor and the position detecting sensor, without receiving an additional input from the user, and based on this determination, may control the operation of the heater. As a result, the aerosol generating device may reduce time taken for the heater to reach a target temperature, by initiating the operation of the heater based on the determination that the cigarette is accommodated (or inserted), and may prevent unnecessary power consumption and mal-function of the heater, by stopping the operation of the heater based on the determination that the cigarette is removed from the aerosol generating device.

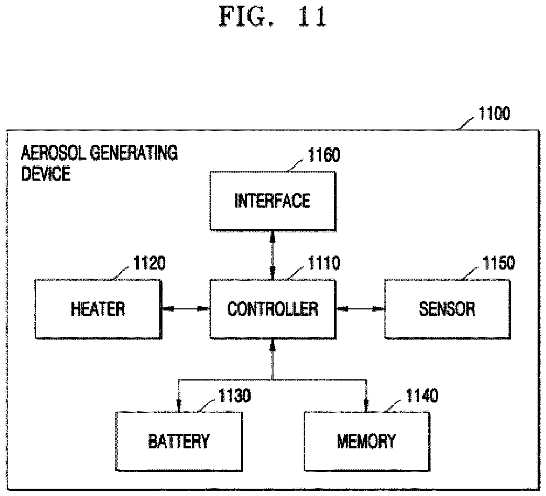

FIG. 11 is a block diagram of hardware components of an aerosol generating device 1100.

Referring to FIG. 11, the aerosol generating device 1100 may include a heater 1120, a controller 1110, a memory 1140, a battery 1130, a sensor 1150, and an interface 1160.

The heater 1120 may be electrically heated via power supplied from the battery 1130 under control of the controller 1110. When the cigarette 3 moves along the accommodation passage 1004h and an end of the cigarette 3 reaches the bottom wall 1004b of the accommodation portion 1004, the heater 1120 may be located inside the cigarette 3. Thus, the heater 1120 that is heated may increase a temperature of an aerosol generating material inside the cigarette 3. The heater 1120 may include all heaters which may be inserted into a cigarette. Also, only a portion of the heater 1120 may be heated.

The heater 1120 may include an electro-resistive heater. For example, the heater 1120 may include an electrically conductive track and the heater 1120 may be heated when currents flow through the electrically conductive track.

For safe use, power according to the specifications of 3.2 V, 2.4 A, and 8W may be supplied to the heater 1120. However, it is not limited thereto. For example, when power is supplied to the heater 1120, a surface temperature of the heater 1120 may increase to a temperature equal to or greater than 400 °C. The surface temperature of the heater 1120 may increase to about 350 °C before 15 seconds after a point at which power supply to the heater 1120 is started.

An additional temperature detecting sensor may be included in the case 1001. Alternatively, the temperature detecting sensor may not be included in the case 1001 and the heater 1120 may function as the temperature detecting sensor. Alternatively, while the heater 1120 of the case 1001 may function as the temperature detecting sensor, an additional temperature detecting sensor may further be included in the case 1001. In order for the heater 1120 to function as the temperature detecting sensor, the heater 1120 may include at least one electrically conductive track for emitting heat and detecting temperature. Alternatively, the heater 1120 may include a first electrically conductive track for emitting heat and a second electrically conductive track for detecting temperature.

For example, when a voltage supplied to the second electrically conductive track and a current flowing through the second electrically conductive track are measured, a resistance (R) may be determined. Here, a temperature (T) of the second electrically conductive track may be determined based on Equation 1 below.

Equation 1

$$R = R_0(1 + \alpha(T - T_0))$$

In Equation 1, R denotes a current resistance value of the second electrically conductive track, R0 denotes a resistance value at a temperature T0 (for example, 0 °C), and a may denote a resistance temperature coefficient of the second electrically conductive track. The conductive material (for example, metal) has a unique resistance temperature coefficient, and thus, a may be pre-determined according to the conductive material included in the second electrically conductive track. Thus, when the resistance R of the second electrically conductive track is determined, the temperature T of the second electrically conductive track may be calculated based on the Equation 1.

The heater 1120 may include one or more electrically conductive tracks (the first electrically conductive track and the second electrically conductive track). For example, the heater 1120 may include two first electrically conductive tracks together with one second electrically conductive track or two second electrically conductive tracks. However, it is not limited thereto.

The electrically conductive track may include an electro-resistive material. For example, the electrically conductive track may include a metal. As another example, the electrically conductive track may include an electrically conductive ceramic material, carbon, a metal alloy, or a composite of a ceramic material and a metal.

Also, the case 1001 may include both an electrically conductive track functioning as the temperature detecting sensor and the temperature detecting sensor.

The controller 1110 is a hardware component configured to control general operations of the aerosol generating device 1100. The controller 1110 may include an integrated circuit realized as a processing unit, such as a microprocessor, a microcontroller, etc.

The controller 1110 may analyze a result sensed by the sensor 1150 and control subsequent processes. The controller 1110 may initiate or stop the power supply from the battery 1130 to the heater 1120 according to the sensed result. Also, the controller 1110 may control the amount of power supplied to the heater 1120 and the time of the power supply in order to heat the heater 1120 to a certain temperature or keep the heater 1120 at an appropriate temperature. Furthermore, the controller 1110 may process various pieces of input information and output information of the interface 1160.

The controller 1110 may count the number of times of smoking of the user using the aerosol generating device 1100 and control, based on a result of the counting, related functions of the aerosol generating device 1100 to limit the smoking of the user.

The memory 1140 may be a hardware component configured to store various pieces of data processed in the aerosol generating device 1100, and the memory 1140 may store data processed or to be processed by the controller 1110. The memory 1140 may include various types of memories, such as random access memory, such as dynamic random access memory (DRAM), static random access memory (SRAM), etc., read-only memory (ROM), electrically erasable programmable read-only memory (EEPROM), etc.

The memory 1140 may store data about smoking patterns of the user, such as smoking time, the number of times of smoking, etc. Also, the memory 1140 may store data related to the value of the reference temperature change in the case in which the cigarette is accommodated in the accommodation passage.

The battery 1130 may supply power used for the aerosol generating device 1100 to operate. That is, the battery 1130 may supply power to heat the heater 1120. Also, the battery 1130 may supply power required for operations of other hardware components included in the aerosol generating device 1100, such as the controller 1110, the sensor 1150, and the interface 1160. The battery 1130 may include a LiFePO4 battery. However, the battery 1130 is not limited thereto and may include a LiCoO2 battery, a lithium titantate battery, etc. The battery 1130 may include a re-chargeable battery or a disposable battery.

The sensor 1150 may include various types of sensors, such as a puff detecting sensor (a temperature detecting sensor, a flow detecting sensor, a position detecting sensor, etc.), a cigarette insertion detecting sensor, a position detecting sensor of the door 1003, a temperature detecting sensor of the heater 1030, etc. A value obtained as a result of a sensing operation of the sensor 1150 may be provided to the controller 1110 and the controller 1110 may, based on the provided value, control the aerosol generating device 1100 to perform various functions, such as controlling a temperature of the heater, limiting of smoking, determining a position change of the door 1003, determining whether or not the cigarette 3 is inserted, displaying notification, etc.

The interface 1160 may include various interfacing devices, such as a display or a lamp for outputting visual information, a motor for outputting haptic information, a speaker for outputting sound information, input/output (I/O) interfacing devices (for example, a button or a touch screen) for receiving information input from a user or outputting information to the user, terminals for performing data communication or receiving charging power, communication interfacing modules for performing wireless communication (for example, Wi-Fi, Wi-Fi direct, Bluetooth, near-field communication (NFC), etc.) with external devices. However, the aerosol generating device 1100 may be realized by selectively including only one or more of the various examples of the interfacing devices described above.

While the disclosure has been particularly shown and described with reference to embodiments thereof, it will be understood by those of ordinary skill in the art that various changes in form and detail may be made therein without departing from the spirit and scope of the disclosure as defined by the following claims. Thus, the embodiments should be considered in a descriptive sense only and not for purposes of limitation. Therefore, the scope of the disclosure is defined not by the detailed description of the disclosure but by the appended claims, and all differences within the scope will be construed as being included in the disclosure.

It follows a list of examples:

1. An aerosol generating device comprising:

an accommodation passage configured to accommodate a cigarette;
a heater configured to heat the cigarette accommodated in the accommodation passage;
a cigarette insertion detecting sensor located inside the accommodation passage; and
a controller,
wherein
the controller is configured to
detect whether or not the cigarette is inserted into the accommodation passage by using the cigarette insertion detecting sensor.

2. The aerosol generating device of example 1, wherein

the controller is configured to
receive, from the cigarette insertion detecting sensor, a signal of detecting whether or not the cigarette is inserted into the accommodation passage, and control, based on the received signal, operation of the heater.

3. The aerosol generating device of example 1, wherein

the cigarette insertion detecting sensor
is located at a side wall forming the accommodation passage.

4. The aerosol generating device of example 1, wherein
the cigarette insertion detecting sensor comprises at least one of a film sensor, a pressure sensor, an optical sensor, or an infrared sensor.

5. A method, performed by an aerosol generating device, of generating an aerosol by heating a cigarette, the method comprising:

receiving, from a cigarette insertion detecting sensor located inside an accommodation passage, a signal of detecting whether or not the cigarette is inserted into the accommodation passage;
determining, based on the received signal, whether or not the cigarette is inserted into the accommodation passage; and

controlling, based on whether or not the cigarette is inserted into the accommodation passage, operation of a heater configured to heat the cigarette accommodated in the accommodation passage.

6. An aerosol generating device comprising:

an accommodation passage configured to accommodate a cigarette;
a heater configured to heat the cigarette accommodated in the accommodation passage;
a temperature detecting sensor configured to detect a temperature change of the heater; and
a controller,
wherein
the controller is configured to
receive, from the temperature detecting sensor, a signal of detecting the temperature change of the heater and determine, based on the received signal, whether or not the cigarette is accommodated in the accommodation passage.

7. The aerosol generating device of example 6, wherein

the controller is further configured to
control, based on whether or not the cigarette is accommodated in the accommodation passage, operation of the heater.

8. The aerosol generating device of example 7, wherein

the controller is further configured to
determine whether a value of the temperature change of the heater, the value being derived from the received signal, corresponds to a value of a reference temperature change corresponding to a case in which the cigarette is accommodated in the accommodation passage, by comparing the value of the temperature change of the heater with the value of the reference temperature change, and control, based on a result of the determination, the operation of the heater.

9. The aerosol generating device of example 8, wherein

the controller is further configured to
initiate the operation of the heater as a result of determining that the cigarette is accommodated in the accommodation passage when the value of the temperature change of the heater corresponds to the value of the reference temperature change, and stop the operation of the heater as a result of determining that the cigarette is not accommodated in the accommodation passage when the value of the temperature change of the heater does not correspond to the value of the reference temperature change.

10. A method, performed by an aerosol generating device, of generating an aerosol by heating a cigarette, the method comprising:

receiving, from a temperature detecting sensor configured to detect a temperature change of a heater, a signal of detecting the temperature change of the heater;
determining whether a value of the temperature change of the heater, the value being derived from the received signal, corresponds to a value of a reference temperature change corresponding to a case in which the cigarette is accommodated in an accommodation passage, by comparing the value of the temperature change of the heater with the value of the reference temperature change; and
determining, based on a result of the determination, whether or not the cigarette is accommodated in the accommodation passage.

11. The method of example 10,

further comprising
controlling operation of the heater, based on whether or not the cigarette is accommodated in the accommodation passage, wherein
the controlling comprises
initiating the operation of the heater by determining that the cigarette is accommodated in the accommodation passage when the value of the temperature change of the heater corresponds to the value of the reference temperature change, and stopping the operation of the heater as a result of determining that the cigarette is not accommodated in the accommodation passage when the value of the temperature change of the heater does not correspond to the value of the reference temperature change.

12. An aerosol generating device comprising:

a cover;
an accommodation passage configured to accommodate a cigarette inserted through the cover;
a heater configured to heat the cigarette accommodated in the accommodation passage; and
a controller,
wherein
the cover comprises:

a door included in an upper surface of the cover and exposing the accommodation passage to the outside; and a position detecting sensor configured to detect a position change of the door, and the controller is configured to receive, from the position detecting sensor, a signal of detecting the position change of the door and determine, based on the received signal, whether or not the cigarette is inserted into the accommodation passage.

13. The aerosol generating device of example 12, wherein

the controller is further configured to control operation of the heater, based on whether or not the cigarette is inserted into the accommodation passage.

14. The aerosol generating device of example 13, wherein

the controller is further configured to initiate the operation of the heater when the position change of the door, the position change being derived from the received signal, includes a change from a closed position to an open position, and stop the operation of the heater when the position change of the door, the position change being derived from the received signal, includes a change from the open position to the closed position.

15. A method, performed by an aerosol generating device, of generating an aerosol by heating a cigarette, the method comprising:

receiving, from a position detecting sensor configured to detect a position change of a door included in an upper surface of a cover and exposing an accommodation passage configured to accommodate the cigarette to the outside, a signal of detecting the position change of the door; and determining, based on the received signal, whether or not the cigarette is inserted into the accommodation passage.

16. The method of example 15,

further comprising controlling, based on whether or not the cigarette is inserted into the accommodation passage, operation of a heater configured to heat the cigarette accommodated in the accommodation passage, wherein the controlling comprises initiating the operation of the heater when the position change of the door, the position change being derived from the received signal, includes a change from a closed position to an open position, and stopping the operation of the heater when the position change of the door, the position change being derived from the received signal, includes a change from the open position to the closed position.

17. An aerosol generating device comprising:

a cover; an accommodation passage configured to accommodate a cigarette inserted through the cover; a heater configured to heat the cigarette accommodated in the accommodation passage; a temperature sensor configured to detect a temperature change of the heater; and a controller, wherein the cover comprises a door included in an upper surface of the cover and exposing the accommodation passage to the outside; and a position detecting sensor configured to detect a position change of the door, and the controller is configured to receive, from the temperature detecting sensor, a signal of detecting the temperature change of the heater, and from the position detecting sensor, a signal of detecting the position change of the door, and determine, based on the signal received from the temperature detecting sensor and the signal received from the position detecting sensor, whether or not the cigarette is accommodated in the accommodation passage.

18. A method, performed by an aerosol generating device, of generating an aerosol by heating a cigarette, the method comprising:

receiving, from a temperature detecting sensor configured to detect a temperature change of a heater, a signal of detecting the temperature change of the heater; receiving, from a position detecting sensor configured to detect a position change of a door included in an upper surface of a cover and exposing to the outside an accommodation passage configured to accommodate the cigarette , a signal of detecting the position change of the door; and

determining, based on the signal received from the temperature detecting sensor and the signal received from the position detecting sensor, whether or not the cigarette is accommodated in the accommodation passage.

**Claims**

1. An aerosol generating device comprising:

   a cover;
   an accommodation passage configured to accommodate a cigarette inserted through the cover;
   a heater configured to heat the cigarette accommodated in the accommodation passage; and

   a controller,
   wherein the cover comprises:

   a door provided on an upper surface of the cover and configured to expose the accommodation passage to outside;
   a position detecting sensor configured to detect a position change of the door, and wherein the controller is configured to:

   receive a signal of detecting the position change of the door from the position detecting sensor; and
   determine, based on the received signal, whether the cigarette is inserted into the accommodation passage.

2. The aerosol generating device of claim 1, wherein the controller is further configured to control an operation of the heater based on whether or not the cigarette is inserted into the accommodation passage.

3. The aerosol generating device of claim 2, wherein the controller is further configured to:

   initiate the operation of the heater when the position change of the door, the position change being derived from the received signal, indicates a change from a closed position to an open position; and
   stop the operation of the heater when the position change of the door, the position change being derived from the received signal, indicates a change from the open position to the closed position.

4. A method, performed by an aerosol generating device, of generating aerosol by heating a cigarette, the method comprising:

receiving, from a position detecting sensor configured to detect a position change of a door provided on an upper surface of a cover, and expose an accommodation passage for accommodating the cigarette to outside, a signal of detecting the position change of the door; and
determining, based on the received signal, whether the cigarette is inserted into the accommodation passage.

5. The method of claim 4, further comprising controlling, based on whether the cigarette is inserted into the accommodation passage, an operation of a heater configured to heat the cigarette accommodated in the accommodation passage,
wherein the controlling comprises:

   initiating the operation of the heater when the position change of the door, the position change being derived from the received signal, indicates a change from a closed position to an open position; and
   stopping the operation of the heater when the position change of the door, the position change being derived from the received signal, indicates a change from the open position to the closed position.

FIG. 1

FIG. 2

FIG. 3

FIG. 4

# FIG. 5

FIG. 6

# FIG. 7

```
              ( START )
                  │
                  ▼
┌─────────────────────────────────────┐
│ RECEIVE SIGNAL OF DETECTING WHETHER  │
│ OR NOT CIGARETTE IS INSERTED INTO    │── 710
│ ACCOMMODATION PASSAGE FROM CIGARETTE │
│ INSERTION DETECTING SENSOR LOCATED   │
│ IN ACCOMMODATION PASSAGE             │
└─────────────────────────────────────┘
                  │
                  ▼
┌─────────────────────────────────────┐
│ DETERMINE WHETHER OR NOT CIGARETTE   │
│ IS INSERTED INTO ACCOMMODATION       │── 720
│ PASSAGE BASED ON RECEIVED SIGNAL     │
└─────────────────────────────────────┘
                  │
                  ▼
┌─────────────────────────────────────┐
│ CONTROL OPERATION OF HEATER          │
│ CONFIGURED TO HEAT CIGARETTE         │
│ ACCOMMODATED IN ACCOMMODATION        │── 730
│ PASSAGE, BASED ON WHETHER OR NOT     │
│ CIGARETTE IS INSERTED INTO           │
│ ACCOMMODATION PASSAGE                │
└─────────────────────────────────────┘
                  │
                  ▼
              ( END )
```

FIG. 8

# FIG. 9

START

RECEIVE SIGNAL OF DETECTING TEMPERATURE
CHANGE OF HEATER FROM TEMPERATURE
DETECTING SENSOR CONFIGURED TO
DETECT TEMPERATURE CHANGE OF HEATER — 910

DETERMINE WHETHER OR NOT TEMPERATURE
CHANGE OF HEATER CORRESPONDS TO REFERENCE
TEMPERATURE CHANGE BY COMPARING
TEMPERATURE CHANGE OF HEATER DERIVED FROM — 920
RECEIVED SIGNAL WITH REFERENCE TEMPERATURE
CHANGE OF CASE IN WHICH CIGARETTE IS
INSERTED INTO ACCOMMODATION PASSAGE

DETERMINE WHETHER OR NOT CIGARETTE IS
ACCOMMODATED IN ACCOMMODATION PASSAGE — 930
BASED ON RESULT OF DETERMINATION

END

# FIG. 10

START

RECEIVE SIGNAL OF DETECTING POSITION CHANGE
OF DOOR FROM POSITION DETECTING SENSOR
CONFIGURED TO DETECT POSITION CHANGE OF
DOOR PROVIDED ON UPPER SURFACE OF
COVER AND EXPOSING ACCOMMODATION PASSAGE
ACCOMMODATING CIGARETTE — 1010

DETERMINE WHETHER OR NOT CIGARETTE
IS INSERTED INTO ACCOMMODATION
PASSAGE BASED ON RECEIVED SIGNAL — 1020

END

FIG. 11